# EUROPÄISCHE PATENTANMELDUNG

(11) **EP 2 792 385 A1**
(43) Veröffentlichungstag der Anmeldung: **22.10.2014**
(21) Anmeldenummer: 14163712.4
(22) Anmeldetag: 07.04.2014
(51) Int. Cl.: A61N 1/375

(54) **Lösbare Kontaktverbindungseinrichtung für Elektroden an einem elektromedizinischen Gerät**

(30) Priorität: 18.04.2013 US 201361813187 P
(71) Anmelder: BIOTRONIK SE & Co. KG, 12359 Berlin (DE)
(72) Erfinder: Hartmann-Bax, Kathy, 14947 Nuthe-Urstromtal (DE); Ortscheid, Annett, 14612 Falkensee (DE)
(74) Vertreter: Lindner-Vogt, Karin L.

(57) **Zusammenfassung**

Eine lösbare Kontaktverbindungseinrichtung für Elektroden an einem elektromedizinischen Gerät, insbesondere für Elektroden von aktiven Implantaten wie z.B. Neurostimulator-Geräten, umfasst
- ein an der Elektrode (1) vorgesehenes Kontaktende (4) mit mindestens einem elektrischen Kontakt (5),
- einen geräteseitigen Anschlusskopf (7) am elektromedizinischen Gerät (2),
- eine Anschlussöffnung (6) am Anschlusskopf (7) zur Verankerung des Kontaktendes (4) der Elektrode (1) unter Herstellung einer elektrischen Kontaktverbindung und lösbaren mechanischen Fixierung, und
- eine Knickschutzeinrichtung (K) am Austritt des Kontaktendes (4) aus der Anschlussöffnung (6),
- wobei die Knickschutzeinrichtung (K) eine vor dem mindestens einen elektrischen Kontakt (5) auf dem Kontaktende (4) der Elektrode (1) sitzende, flexible Knickschutz-Hülse (8) aufweist, die mit ihrem dem Anschlusskopf (7) zugewandten Fixierende (9) in eine gegenüber der Anschlussöffnung (6) erweiterten Aufnahme-Aussparung (12) eingeschoben und befestigt ist.

## Beschreibung

Die Erfindung betrifft eine lösbare Kontaktverbindungseinrichtung für Elektroden an einem elektromedizinischen Gerät, insbesondere für Elektroden von implantierbaren Neurostimulator-Geräten, mit den im Oberbegriff des Patentanspruches 1 angegebenen Merkmalen.

Derartige lösbare Kontaktverbindungseinrichtungen umfassen üblicherweise ein an der Elektrode vorgesehenes Kontaktende mit mindestens einem elektrischen Kontakt, einen geräteseitigen Anschlusskopf am elektromedizinischen Gerät, eine Anschlussöffnung am Anschlusskopf zur Verankerung des Kontaktendes der Elektrode unter Herstellung einer elektrischen Kontaktverbindung und lösbaren mechanischen Fixierung, sowie eine Knickschutzeinrichtung am Austritt des Kontaktendes aus der Anschlussöffnung.

Zum Hintergrund der Erfindung ist festzuhalten, dass derartige lösbare Kontaktverbindungseinrichtungen, besonders wenn sie bei Elektroden für Neurostimulatoren eingesetzt werden, einen sogenannten "360°-Knickschutz" am Übergang vom geräteseitigen Anschluss zur Elektrode benötigen, da die Elektrode solcher elektromedizinischer Geräte seitlich praktisch in alle Raumrichtungen aus der Längsachse des Anschlussendes ausgelenkt werden kann.

Die in diesem Zusammenhang notwendige Knickschutzeinrichtung ist aus dem Stand der Technik gemäß offenkundiger Vorbenutzung seitens der Anmelderin grundsätzlich bekannt. Sie besteht aus einem um die Mündung der Anschlussöffnung am Anschlusskopf des elektromedizinischen Gerätes angeformten Stutzen in Kombination mit einer auf der Elektrode verschiebbaren Knickschutztülle. Auch ein aus dem Anschlusskopf herausragendes Schlauchstück ist bekannt und erfüllt den Zweck einer Knickschutzmaßnahme.

Diese bekannten Lösungen haben den Nachteil, dass sie von der an sich glattflächigen Oberfläche des Anschlusskopfes vorspringen, was ein Nacharbeiten der Oberflächen des Anschlusskopfes, wie beispielsweise ein Entgraten und Polieren, nicht erlaubt. Der Grund hierfür liegt in erster Linie darin, dass diese Anschlussköpfe zur Ausbildung dieser Knickschutzeinrichtungen in der Regel komplett aus Vergussharz gebildet sind, was eine Oberflächenveredelung zur Erfüllung hoher Qualitätsansprüche erschwert.

Ausgehend von der geschilderten Problematik des Standes der Technik liegt der Erfindung die Aufgabe zugrunde, eine Knickschutzeinrichtung an einer gattungsgemäßen lösbaren Kontaktverbindungseinrichtung zu schaffen, die eine unabhängige Nachbearbeitung des Anschlusskopfes des elektromedizinischen Gerätes bei hoher Knickschutzwirkung erlaubt.

Die Aufgabe wird durch die im Kennzeichnungsteil des Anspruches 1 angegebenen Merkmale gelöst. Demnach weist die Knickschutzeinrichtung eine vor dem mindestens einen elektrischen Kontakt auf dem Kontaktende der Elektrode sitzende, flexible, also biegsame Knickschutz-Hülse auf, die mit ihrem dem Anschlusskopf zugewandten Fixierende in eine gegenüber der Anschlussöffnung erweiterten Aufnahme-Aussparung eingeschoben befestigt ist.

Grundkonzept der Erfindung ist also die räumliche Trennung der Knickschutzeinrichtung vom Anschlusskopf, der lediglich eine entsprechende Aufnahme-Aussparung für das Fixierende der Knickschutz-Hülse aufweisen muss. Da diese Aussparung keinen Vorsprung an der Oberfläche des Anschlusskopfes, sondern lediglich eine Vertiefung oder Einbuchtung bildet, die bei angeschlossener Elektrode ohnehin durch das Fixierende der Knickschutz-hülse geschlossen ist, können am Anschlusskopf problemlos Nacharbeiten der Oberfläche vorgenommen und der Anschlusskopf selbst aus einem hinsichtlich seiner Oberflächengüte ausgewählten Material bestehen. Die Verwendung eines Vergussharzes für die Herstellung des Anschlusskopfes ist wegen des weggefallenen Tüllen- oder Schlauchvorsprungs am Anschlusskopf problemlos möglich. Gleichwohl wird durch die Knickschutz-Hülse ein wirkungsvoller 360°-Schutz am Übergang zum Anschlusskopf gewährleistet, der auch größeren Kräften und Muskelbewegungen, wie sie gerade im Umfeld von aktiven Implantaten wie z.B. Neurostimulator-Geräten auftreten können, widersteht.

In den abhängigen Ansprüchen sind bevorzugte Weiterbildungen des Erfindungsgegenstandes angegeben. So kann es vorgesehen sein, dass die in Längsaxialrichtung weisende Kopffläche der Knickschutz-Hülse als Anschlagfläche mit der Bodenfläche der erweiterten Aufnahme-Aussparung kooperiert. Damit bildet die Knickschutz-Hülse gleichzeitig den Anschlag für das Einschieben des Kontaktendes der Elektrode in die Anschlussöffnung des Gerätes. Damit ist die Einstecktiefe des Anschlussendes festgelegt.

Gemäß einer bevorzugten Weiterbildung ist es vorgesehen, dass die Knickschutz-Hülse in einem Abstand von ihrer Kopffläche, der der Tiefe der Aufnahme-Aussparung entspricht, mit einer Markierung versehen ist. Damit ist eine optische Kontrollmöglichkeit für die Positionierung der Elektrode im angeschlossenen Zustand gegeben.

Vorzugsweise wird die Markierung durch eine in Peripherrichtung umlaufende Ringnut in der Knickschutz-Hülse gebildet. Damit kann auch "blind" mit Hilfe des Tastsinns die Positionierung des Kontaktendes der Elektrode in der Anschlussöffnung erfühlt werden.

Die gemäß einer weiteren bevorzugten Ausführungsform mögliche kegelstumpfförmige, sich in Richtung der Elektrode verjüngende Gestalt der Knickschutz-Hülse unterstützt die schonende Behandlung der Elektrode bei ihrer Auslenkung aus der Axialrichtung der Anschlussöffnung im Anschlusskopf.

Eine herstellungstechnisch einfache und materialtechnisch vorteilhafte Weiterbildung sieht die Herstellung der Knickschutz-Hülse aus einem vollvolumigen, spritzgegossenen, flexiblen biokompatiblen Elastomer vorzugsweise in Kombination mit mindestens einem Stützelement aus Keramik, Metall oder Kunststoff vor.

Für die Anordnung der Knickschutz-Hülse auf der Elektrode sind zwei grundsätzliche Alternativen möglich, nämlich einerseits eine ortsfeste Fixierung der Knickschutz-Hülse auf dem Kontaktende der Elektrode. Dadurch ist eine saubere und exakte Zuordnung der Position der Knickschutz-Hülse zum Kontaktende und damit auch relativ zur Anschlussöffnung möglich, so dass die Anschlagfunktion und Positionierungskontrolle durch das Einschieben der Knickschutz-Hülse in die Aussparung der Aufnahmeöffnung besonders zuverlässig möglich sind.

Herstellungstechnisch einfach ist das gemäß einer weiteren bevorzugten Ausführungsform vorgesehene Anspritzen der Knickschutz-Hülse direkt an das Kontaktende der Elektrode.

Auch ein Aufschweißen oder Aufkleben sind als Variante davon möglich, wobei dann in letzterem Fall ein biokompatibler Klebstoff vorzuziehen ist.

Als Alternative zu der festen Verbindung zwischen Knickschutz-Hülse und Elektrode kann auch deren verschiebbare Lagerung auf der Elektrode vorgesehen sein.

Gemäß einer weiteren bevorzugten Ausführungsform kann die längsaxial verlaufende Hülsenöffnung der Knickschutz-Hülse durch mindestens ein Dichtelement gegenüber dem Kontaktende der Elektrode abgedichtet sein. Damit können axiale und radiale Dichtfunktionen durch die Knickschutz-Hülse übernommen werden. Für die Ausbildung des Dichtelementes können übliche Konfigurationen, wie ein Dichtring, eine ballige Dichtfläche oder eine Dichtlippe vorgesehen werden. Wenn die Knickschutz-Hülle aus einem flexiblen Material, wie beispielsweise aus Silikon gefertigt ist, sind derartige Dichtelemente problemlos an der Knickschutz-Hülse auszubilden.

Diese flexible Ausbildung ist im Übrigen auch im Zusammenhang mit der Verschiebbarkeit der Knickschutz-Hülse auf der Elektrode von Vorteil. Durch eine insbesondere radial nach innen flexible Spezifikation des Materials der Knickschutz-Hülse kann ein Anwender diese zwischen Daumen und Zeigefinger zusammenpressen, womit eine temporäre Fixierung der Hülse auf der Elektrodenleitung erfolgt, die durch Loslassen wieder gelöst werden kann.

Eine weitere bevorzugte Dichtungsmaßnahme kann darin bestehen, auf der Außenseite des Fixierendes der Knickschutz-Hülse eine umlaufende Dichtschulter vorzusehen, die mit der Innenwand der Aufnahme-Aussparung kooperiert und damit die Aussparung hermetisch abdichtet.

Ferner kann vorgesehen sein, zur weiter verbesserten Fixierung der Knickschutz-Hülse in der Aussparung eine Befestigungseinrichtung insbesondere in Form einer Verrastung zwischen beiden Bauteilen vorzusehen, die durch eine am Fixierende umlaufende Befestigungsschulter und eine entsprechende Ringnut in der Innenwand der Aussparung gebildet sein kann. In der Montagestellung der Knickschutz-Hülse rastet diese Befestigungsschulter dann in die Ringnut ein und sorgt für einen sicheren Halt.

Schließlich liegt eine weitere bevorzugte Weiterbildung der Kontaktverbindungseinrichtung darin, die Mündung der Anschlussöffnung und/oder die Kopffläche der Knickschutz-Hülse mit einer umlaufenden Fase zu versehen. Diese Anfasung bewirkt eine Selbstzentrierung der Knickschutz-Hülse beim ihrem Einführen in die Aussparung im Anschlusskopf. Die Anwendung des elektromedizinischen Gerätes wird damit erleichtert.

Weitere Merkmale, Einzelheiten und Vorteile der Erfindung ergeben sich aus der nachfolgenden Beschreibung eines Ausführungsbeispiels anhand der beigefügten Zeichnungen. Es zeigen:
- Fig. 1: eine perspektivische Teilansicht einer Elektrode im Bereich ihres Kontaktendes mit Knickschutz-Hülse,
- Fig. 2: eine perspektivische Teilansicht einer Elektrode analog Fig. 1 mit einem elektromedizinischen Gerät vor dem Einführen des Kontaktendes der Elektrode in den Anschlusskopf des Gerätes,
- Fig. 3: eine perspektivische Teildarstellung von Elektrode und Gerät mit Anschlusskopf im kontaktierten Zustand,
- Fig. 4: eine perspektivische Teilansicht einer Elektrode im Bereich ihres Kontaktendes mit Knickschutz-Hülse in einer zweiten Ausführungsform,
- Fig. 5: eine perspektivische Teilansicht einer Elektrode analog Fig. 4 mit einem elektromedizinischen Gerät vor dem Einführen des Kontaktendes der Elektrode in den Anschlusskopf des Gerätes,
- Fig. 6: eine perspektivische Darstellung der Elektrode gemäß Fig. 4 und 5 sowie eines elektromedizinischen Geräts mit dem Anschlusskopf im kontaktierten Zustand, und
- Fig. 7: einen perspektivischen Längsaxialschnitt der bei den Elektroden gemäß Fig. 4 bis 6 verwendeten Knickschutz-Hülse.

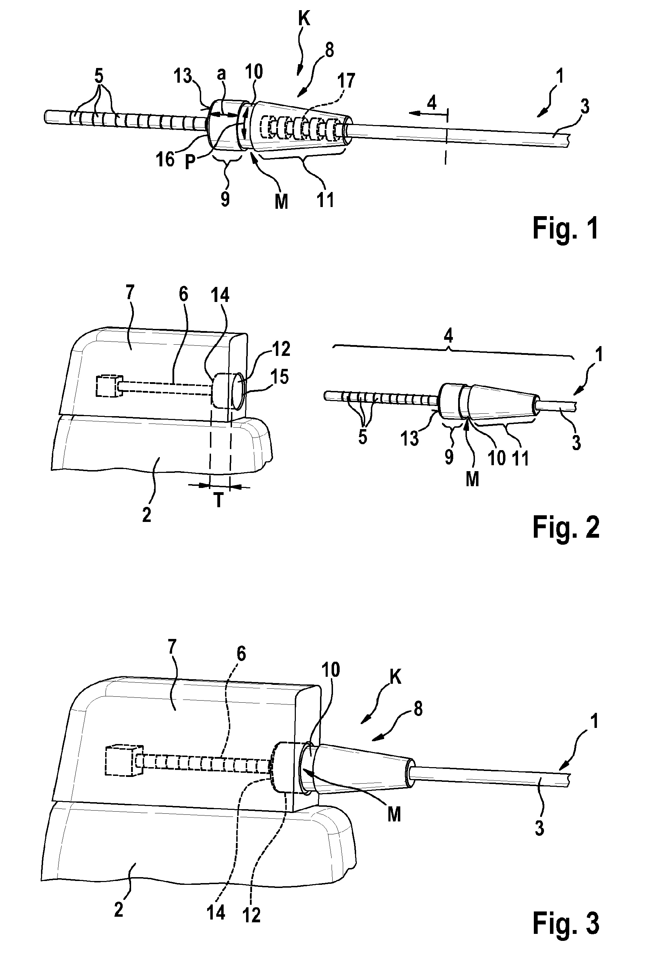

Wie aus den Fig. 1 bis 3 deutlich wird, weist eine Elektrode 1 beispielsweise eines implantierbaren (Neurostimulator) -Gerätes 2 (siehe Fig. 2 und 3) einen isolierenden schlauchförmigen Elektrodenkörper 3 mit einem Kontaktende 4 auf, an dem mehrere ringförmige elektrische Kontakte 5 angeordnet sind.

Diese kooperieren mit entsprechenden Gegenkontakten (nicht dargestellt) im Anschlusskopf 7 des Gerätes 2, die mit der elektronischen Gerätesteuerung des Geräts 2 entsprechend verschaltet sind.

Zum Schutz der Elektrode 1 gegen dynamische Bewegungen, Biegemomente und dergleichen ist eine als Ganzes mit K bezeichnete Knickschutzeinrichtung am Austritt des Kontaktendes 4 aus der Anschlussöffnung 6 vorgesehen, die im gezeigten Ausführungsbeispiel durch eine Knickschutz-Hülse 8 realisiert ist. Diese besteht aus einem flexiblen biokompatiblen Elastomer und ist direkt an den Elektrodenkörper 3 angespritzt. Der Hülsenkörper gliedert sich dabei in ein den Kontakten 5 am Kontaktende 4 zugewandtes Fixierende 9, eine in Peripherrichtung P umlaufende Ringnut 10 und einen sich kegelstumpfförmig verjüngenden Tüllenbereich 11. Das zylindrisch ausgebildete Fixierende 9 der Knickschutz-Hülse 8 hat als Gegenpart eine innenzylindrische Aufnahme-Aussparung 12, die die Mündung der Anschlussöffnung 6 bildet und koaxial dazu ausgerichtet ist. Der Abstand a der Ringnut 10 von der Kopffläche 13 des Fixierendes 9 entspricht der Tiefe T der Aufnahme-Aussparung 12. Wird das Kontaktende 4 der Elektrode 1 in die Anschlussöffnung 6 eingeschoben, wirkt die Kopffläche 13 mit dem Boden 14 der Aussparung 12 als Anschlag zusammen, so dass die Ringnut 10 dann als eine optische und ertastbare Markierung M für die korrekte Positionierung des Kontaktendes 4 der Elektrode 1 in der Anschlussöffnung 6 fungiert, wie dies anhand von Fig. 3 gut erkennbar ist. Die Ringnut 10 kann auch erhaben als Ringschulter ausgeführt sein. Ebenso ist eine optische Markierung als Bedruckung denkbar.

Schließlich geht aus Fig. 1 und 2 hervor, dass sowohl die Kopffläche 13 an ihrem Rand als auch die Mündung der Anschlussöffnung 6 mit einer Fase 15 bzw. 16 versehen sind, durch die das Einführen des Fixierendes 9 der Knickschutz-Hülse 8 in die Aussparung 12 erleichtert und eine Selbstzentrierung dieser beiden Komponenten zueinander stattfindet.

Wie in Fig. 1 gestrichelt dargestellt ist, kann in die Knickschutz-Hülse 8 ein Einleger 17 aus Kunststoff-, Metall-, Elastomer- oder Keramik zur Verstärkung der Hülse eingebettet sein.

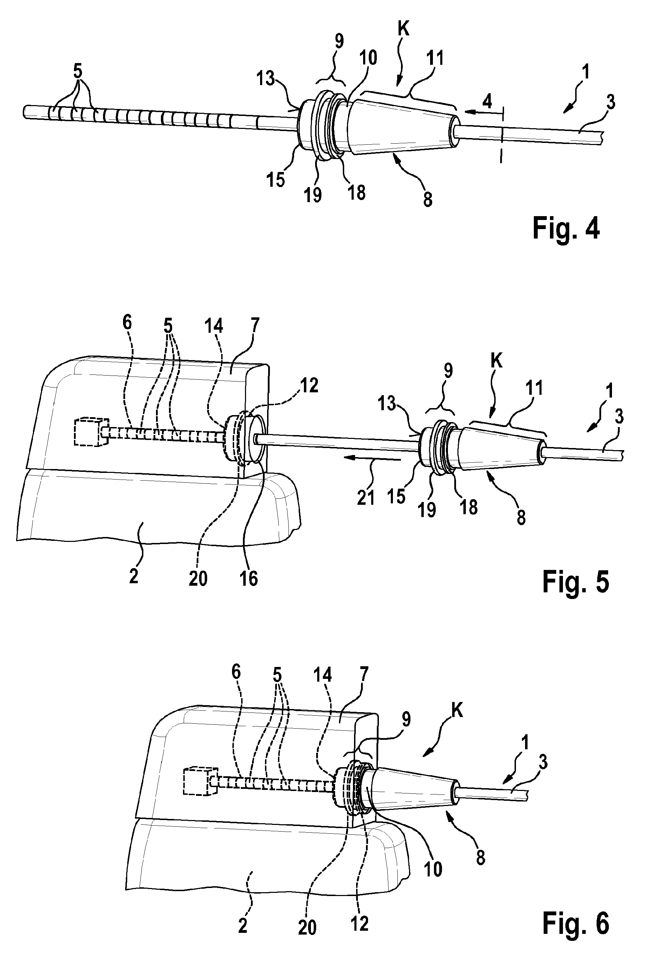

Die in den Fig. 4 bis 7 gezeigte Ausführungsform der Elektrode 1 entspricht in der grundsätzlichen Ausführung der Variante gemäß den Fig. 1 bis 3. Insoweit sind übereinstimmende Komponenten mit identischen Bezugszeichen versehen und bedürfen keiner nochmaligen Erläuterung. Zur Vermeidung von Wiederholungen wird auf die entsprechende Beschreibung der Fig. 1 bis 3 verwiesen.
Wesentliche Unterschiede der Ausführungsform gemäß Fig. 4 bis 7 liegt in der zusätzlichen Anbringung von Dichtungs- und Befestigungselementen am Fixierende 9 der Knickschutz-Hülse 8 sowie in der Aufnahme-Aussparung 12 des Anschlusskopfes 7. Wie in diesem Zusammenhang deutlich wird, ist im Bereich des Fixierendes 9 eine umlaufende Dichtschulter 18 als Dichtungselement vorgesehen, die mit dem gegenüber dem Außendurchmesser der Dichtschulter 18 etwas geringeren Innendurchmesser der Aussparung 12 kooperiert und beim Einschieben der Knickschutz-Hülse 8 in die Aussparung 12 für eine entsprechende Abdichtung sorgt.

Ferner ist in Richtung zum Kontaktende 4 der Elektrode 1 hin vor der Dichtschulter 18 eine im Außendurchmesser und Querschnitt größere Befestigungsschulter 19 angeformt, die als in Umfangsrichtung umlaufender Ringvorsprung ausgebildet ist. Diese Befestigungsschulter 19 kooperiert mit einer entsprechenden Ringnut 20 in der Innenwand der Aussparung 12. Nach dem Einschieben der Knickschutz-Hülse 8 in die Aussparung 12 rastet die Befestigungsschulter 19 in die Ringnut 20 ein, wodurch eine besonders stabile Verankerung der Knickschutz-Hülse 8 im Anschlusskopf 7 des Geräts 2 realisiert wird.

Wie aus einer Zusammenschau der Fig. 5 und 6 hervorgeht, ist hier eine auf dem Elektrodenkörper 3 verschiebbare Version der Knickschutz-Hülse 8 gezeigt. In Fig. 5 ist nämlich die Elektrode 1 mit ihrem Kontaktende 4 bereits in die Anschlussöffnung 6 des Anschlusskopfes 7 in die Endstellung eingeführt. Die Knickschutz-Hülse 8 befindet sich noch mit einem deutlichen Abstand vor dem Anschlusskopf 7.

Um die Elektrode 1 in diese in Fig. 5 gezeigte Position zu verbringen, kann durch die flexible Auslegung der Knickschutz-Hülse 8 der Anwender die Knickschutz-Hülse 8 zwischen Daumen und Zeigefinger einklemmen, wodurch diese temporär auf dem Elektrodenkörper fixiert wird. Die Knickschutz-Hülse 8 wirkt also quasi als verdickter Griff für den Elektrodenkörper 3, der damit bequem in die Anschlussöffnung 6 eingeführt werden kann. Durch Loslassen der Knickschutz-Hülse 8 entspannt sich diese wieder und kann - wie durch den Pfeil 21 angedeutet ist - auf dem Elektrodenkörper 3 in Richtung zur Aussparung 12 verschoben und in die in Fig. 6 gezeigte Endmontageposition verbracht werden.

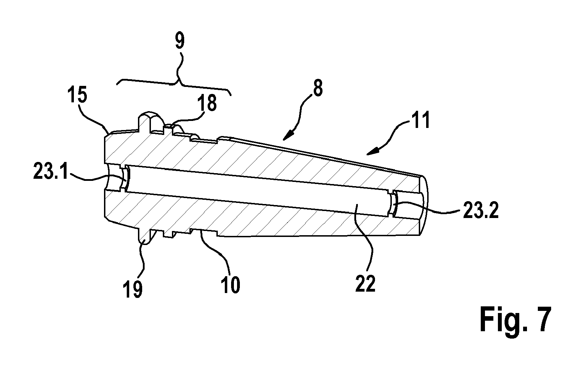

Um die in Fig. 7 erkennbare, längsaxial verlaufende Hülsenöffnung 22 gegenüber dem Elektrodenkörper 3 abzudichten, weist diese vor den beiden Enden jeweils eine nach innen abstehende Dichtlippe 23.1, 23.2 auf, die sich an der Außenfläche des Elektrodenkörpers 3 hermetisch dicht abstützen.

## Patentansprüche

1. Lösbare Kontaktverbindungseinrichtung für Elektroden an einem elektromedizinischen Gerät, insbesondere für Elektroden von aktiven Implantaten, wie Neurostimulator-Geräten, umfassend
- ein an der Elektrode (1) vorgesehenes Kontaktende (4) mit mindestens einem elektrischen Kontakt (5),
- einen geräteseitigen Anschlusskopf (7) am elektromedizinischen Gerät (2),
- eine Anschlussöffnung (6) am Anschlusskopf (7) zur Verankerung des Kontaktendes (4) der Elektrode (1) unter Herstellung einer elektrischen Kontaktverbindung und lösbaren mechanischen Fixierung, und
- eine Knickschutzeinrichtung (K) am Austritt des Kontaktendes (4) aus der Anschlussöffnung (6),
**dadurch gekennzeichnet, dass**
- die Knickschutzeinrichtung (K) eine vor dem mindestens einen elektrischen Kontakt (5) auf dem Kontaktende (4) der Elektrode (1) sitzende, flexible Knickschutz-Hülse (8) aufweist, die mit ihrem dem Anschlusskopf (7) zugewandten Fixierende (9) in eine gegenüber der Anschlussöffnung (6) erweiterten Aufnahme-Aussparung (12) eingeschoben befestigt ist.

2. Kontaktverbindungseinrichtung nach Anspruch 1, **dadurch gekennzeichnet, dass** die in Längsaxialrichtung weisende Kopffläche (13) des Fixierendes (9) der Knickschutz-Hülse (8) als Anschlagfläche mit der Bodenfläche (14) der erweiterten Aufnahme-Aussparung (12) kooperiert.

3. Kontaktverbindungseinrichtung nach Anspruch 2, **dadurch gekennzeichnet, dass** die Knickschutz-Hülse (8) in einem Abstand (a) von ihrer Kopffläche (13), der der Tiefe (T) der Aufnahme-Aussparung (12) entspricht, mit einer Markierung (M) versehen ist.

4. Kontaktverbindungseinrichtung nach Anspruch 3, **dadurch gekennzeichnet, dass** die Markierung (M) durch eine in Peripherrichtung (P) umlaufende Ringnut (10), Ringschulter oder optische Markierung insbesondere in Form eines Aufdrucks in oder an der Knickschutz-Hülse (8) gebildet ist.

5. Kontaktverbindungseinrichtung nach einem der vorgenannten Ansprüche, **dadurch gekennzeichnet, dass** die Knickschutz-Hülse (8) außer im Bereich des Fixierendes (9) und gegebenenfalls der Ringnut (10) eine kegelstumpfförmige, sich in Richtung der Elektrode (1) verjüngende Gestalt und/oder eine zylindrische Gestalt mit Aussparungen zu ihrer Flexibilisierung aufweist.

6. Kontaktverbindungseinrichtung nach einem der vorgenannten Ansprüche, **dadurch gekennzeichnet, dass** die Knickschutz-Hülse (8) aus einem vollvolumigen, spritzgegossenen, flexiblen biokompatiblen Elastomer vorzugsweise in Kombination mit mindestens einem Stützelement aus Keramik, Metall oder Kunststoff besteht.

7. Kontaktverbindungseinrichtung nach einem der vorgenannten Ansprüche, **dadurch gekennzeichnet, dass** die Knickschutz-Hülse (8) ortsfest auf dem Kontaktende (4) der Elektrode (1) fixiert ist.

8. Kontaktverbindungseinrichtung nach Anspruch 6 und 7, **dadurch gekennzeichnet, dass** die Knickschutz-Hülse (8) direkt an das Kontaktende (4) der Elektrode (1) angespritzt ist.

9. Kontaktverbindungseinrichtung nach Anspruch 7, **dadurch gekennzeichnet, dass** die Knickschutz-Hülse (8) auf dem Kontaktende (4) der Elektrode (1) verschweißt oder vorzugsweise mit Hilfe eines biokompatiblen Klebstoffes aufgeklebt ist.

10. Kontaktverbindungseinrichtung nach einem der Ansprüche 1 bis 6, **dadurch gekennzeichnet, dass** die Knickschutz-Hülse (8) verschiebbar auf dem Kontaktende (4) der Elektrode (1) gelagert ist.

11. Kontaktverbindungseinrichtung nach Anspruch 10, **dadurch gekennzeichnet, dass** die Knickschutz-Hülse (8) eine längsaxial verlaufende Hülsenöffnung (22) aufweist, die durch mindestens ein Dichtelement (23.1, 23.2) abgedichtet ist.

12. Kontaktverbindungseinrichtung nach Anspruch 10, **dadurch gekennzeichnet, dass** das Dichtelement durch einen Dichtring, ballige Dichtfläche oder eine Dichtlippe (23.1, 23.2) gebildet ist.

13. Kontaktverbindungseinrichtung nach einem der vorgenannten Ansprüche, **dadurch gekennzeichnet, dass** das Fixierende (9) der Knickschutz-Hülse (8) mit einem mit der Aufnahme-Aussparung (12) kooperierenden Dichtelement, insbesondere einer umlaufenden Dichtschulter (18) und/oder mit einem mit der Aufnahme-Aussparung (12) kooperierenden Befestigungselement, insbesondere einer Befestigungsschulter (19) zur Verrastung mit einer Ringnut (20) in der Aufnahme-Aussparung (12) versehen ist

14. Kontaktverbindungseinrichtung nach einem der vorgenannten Ansprüche, **dadurch gekennzeichnet, dass** die Mündung der Anschlussöffnung (6) und/oder die Kopffläche (13) der Knickschutz-Hülse (8) mit einer Zentrierungshilfe, insbesondere einer umlaufenden Fase (15, 16), versehen sind.

15. Kontaktverbindungseinrichtung nach einem der vorgenannten Ansprüche, **dadurch gekennzeichnet, dass** in die Knickschutz-Hülse (8) ein Einleger (17) zur Verstärkung eingebettet ist.
